# EUROPEAN PATENT SPECIFICATION

(11) **EP 1 476 088 B1**
(45) Date of publication and mention of the grant of the patent: **25.06.2008**
(21) Application number: 02768858.9
(22) Date of filing: 17.09.2002
(51) Int. Cl.: A61B 17/32

(54) **SURGICAL ROTARY ABRADER**
CHIRURGISCHES ROTIERENDES ABRASIONSGERÄT
APPAREIL CHIRURGICAL D'ABRASION ROTATIF

(30) Priority: 17.09.2001 US 322815 P; 17.09.2001 US 322855 P; 17.09.2001 US 322856 P; 17.09.2001 US 322857 P; 17.09.2001 US 322858 P; 16.05.2002 US 380999 P
(43) Date of publication of application: 17.11.2004
(73) Proprietor: HydroCision, Inc., Billerica, MA 01821 (US)
(72) Inventor: MOUTAFIS, Timothy, E., Gloucester, MA 01930 (US); CONNOR, Brian, G., Newfields, New Hampshire 03856 (US)
(74) Representative: HOFFMANN EITLE
(86) International application number: PCT/US2002/029460
(87) International publication number: WO 2003/024340

(56) References cited:
- WO-A-01/22890
- WO-A-01/50965
- DE-A- 2 933 266
- US-A- 4 512 344
- US-A- 6 036 698

## Description

### FIELD OF THE INVENTION

This invention relates to instruments with rotating components for cutting, abrading, polishing, or removing tissue in a surgical procedure. Instruments according to the invention comprise a rotating component connected to a rotatable shaft at least partially enclosed by a sheath.

### BACKGROUND

Tissue contacting components, such as burrs, cutters, abraders, and polishers (referred to collectively herein as "burrs") that are driven by rotating shafts are known. In the case of an open surgical field, uncovered, power driven burrs may be used because, typically, no sheath is required to protect adjacent tissue from the rotating burr or shaft. In endoscopic surgery, however, and in other surgery in tight or confined spaces in the body, it is typically important to provide a sheath or a similar device to shield the burr and, in some cases, the shaft, from contact with the tissue, so that tissue near the intended operative site is not inadvertently removed or damaged. When operating on soft tissue, little force applied to the tissue by the burr is typically needed to effect tissue removal, and movement of the burr towards the sheath, for example by bending of the burr's drive shaft, has not been appreciated as being a concern. When a burr is used on harder tissue such as bone or cartilage, however, the levels of lateral forces (i.e., forces in a direction perpendicular to the longitudinal axis of the shaft) that need to be applied to the burr can force the burr against the sheath in many prior art handpiece configurations. This can potentially damage the burr, as well as the sheath, and can create undesirable shavings or fragments of one or both.

Prior art attempts to address this problem, while potentially suitable at moderate speeds of operation, for example 100 to 8,000 revolutions per minute ("rpm"), are not well suited for use with high-speed burrs, e.g. those operating in excess of the above range, such as those operating at tens of thousands of rpm. One problem is that simple slip bearings which are positioned between the shaft and the sheath and used as support elements in some prior art designs can cause substantial friction at higher speeds, while known support elements resuming in lower friction are prohibitively expensive.

WO 01/22890 A1 discloses the features of the pre-characterising part of claim 1 below. It discloses a surgical instrument including an outer tube with an open distal end and a first radially extending surface. An inner member positioned to move within the outer tube has a surgical tool and a second radially extending surface. These surfaces are positioned to contact each other to maintain the surgical tool within the central bore if the surgical tool separates from the inner member. Furthermore, the first radially extending surface is an inwardly extending circumferential ridge which (despite a small gap between the ridge and the tool when centered within a central bore) acts as a bearing surface on which the surgical tool rotates.

### SUMMARY OF THE INVENTION

We have found that the above-described and other difficulties can be circumvented or mitigated by configuring a surgical instrument with a rotating component that can operate at high speeds on hard tissue without undesirable contact between the burr and the shaft of the instrument, yet can have fewer parts than typical prior art designs.

The invention is defined as claimed in claim 1, the dependent claims are described as embodiments.

In one embodiment, the invention comprises a body having a distal end and a proximal end, a shaft rotatably supported by the body and extending from the distal end of the body, a tissue contacting component drivable by the shaft, and an outer tube surrounding at least a portion of the shaft, wherein the outer tube is constructed and arranged such that, upon application of lateral force to the tissue contacting component, the outer tube contacts the shaft before contacting the tissue contacting component.

Another embodiment comprises a body having a distal end and a proximal end, a shaft rotatably supported by the body and extending from the distal end of the body, a tissue contacting component drivable by the shaft, an outer tube flexibly connected to the distal end of the body and surrounding at least a portion of the shaft, and at least one stand-off constructed and arranged such that, upon application of a lateral force to the tissue contacting component, the at least one stand-off contacts the shaft before the tissue contacting component contacts the outer tube.

Another embodiment of the invention comprises a body having a distal end and a proximal end, a shaft rotatably supported by the body and extending from the distal end of the body, a tissue contacting component drivable by the shaft, a flexible connector member positioned at the distal end of the body, and an outer tube connected to the flexible connector member and surrounding at least a portion of the shaft.

Another embodiment of the invention comprises a body having a distal end and a proximal end, a shaft rotatably supported by the body and extending from the distal end of the body, a tissue contacting component drivable by the shaft, and an outer tube connected to the distal end of the body and surrounding at least a portion of the shaft, wherein the outer tube is more flexible than the shaft.

Another aspect of the invention involves shaft assemblies for use in surgical instruments. One embodiment comprises a rotatable shaft, a tissue contacting component drivable by the shaft, and an outer tube surrounding at least a portion of the shaft, wherein a minimum separation between the tissue contacting component and the outer tube is greater than a minimum separation between the shaft and the outer tube.

The invention is useful in surgical methods. These can involve providing a surgical instrument including a body having a distal end and a proximal end, a shaft roatably supported by the body and extending from the distal end of the body, a tissue contacting component drivable by the shaft, and an outer tube positioned to surround at least a portion of the shaft and tissue contacting component and having an inner surface normally radially spaced from an outer surface of the shaft; contacting the tissue contacting component with tissue of a patient; applying a force to the tissue via the shaft and tissue contacting component, at least a portion the force being laterally directed with respect to the shaft; and laterally displacing at least a portion of the shaft with respect to the outer tube in response to application of the force, thereby decreasing a radial spacing between the outer surface of the shaft and the inner surface of the outer tube without contact between the inner surface of the outer tube and the tissue contacting component.

Use of the instrument of the invention can involve providing the instrument ; contacting the tissue contacting component with tissue of a patient; applying a force to the tissue via the shaft and tissue contacting component, at least a portion the force being laterally directed with respect to the shaft; and laterally displacing at least a portion of the shaft with respect to the outer tube in response to application of the force, thereby decreasing a radial spacing between the outer surface of the shaft and the inner surface of the outer tube without contact between the inner surface of the outer tube and the tissue contacting component.

### BRIEF DESCRIPTION OF THE DRAWINGS

Other advantages, novel features, and uses of the invention will become more apparent from the following detailed description of the invention when considered in conjunction with the accompanying drawings, in which:
FIG. 1 is a plan view of a surgical instrument according to one aspect of the invention;
FIG. 2a is a cross-sectional view of the instrument of FIG. 1, taken along line A-A;
FIG. 2b is a detailed cross-section of the distal end of the device of FIG. 1;
FIG. 2c is a detailed cross-section of the proximal end of the device of FIG. 1;
FIG. 3a is an enlarged view of the distal end of another embodiment of the instrument illustrated in FIG. 1;
FIG. 3b is an enlarged view of the distal end of another embodiment of the instrument illustrated in FIG. 1;
FIG. 3c is an enlarged view of the distal end of another embodiment of the instrument illustrated in FIG. 1;
FIG. 3d is an enlarged cross-sectional view of the distal end of another embodiment of the instrument illustrated in FIG. 1;
FIG. 4 is a partially cut away perspective view of the instrument of FIG. 1;
FIG. 5 is a cross-sectional view of a portion of an alternative embodiment of a surgical instrument according to one aspect of the invention;
FIG. 6a is a diametral cross-sectional view of an alternative embodiment of a surgical instrument according to the invention;
FIG. 6b is a transverse section through the shaft of the FIG 6a embodiment; and FIG7 is a perspective view of a cross-section of an alternative embodiment of a surgical instrument according to the invention,

The drawings are schematic and are not intended to be drawn to scale. In the figures, each identical or substantially similar component that is illustrated in various figures is typically represented by a single numeral or notation. For purposes of clarity, not every component is labeled in every figure, nor is every component of each embodiment of the invention shown where illustration is not necessary to allow those of ordinary skill in the art to understand the invention.

### DETAILED DESCRIPTION

Exemplary embodiments of the invention provide surgical instruments with rotating components suitable for high speed operation, which can also, in some embodiments, be simpler to manufacture than many typical prior art instruments. In the illustrated embodiment of the invention, a surgical instrument with a burr connected to a rotating shaft has a minimum separation between the burr and a surrounding sheath (also referred to herein and in the claims as an "outer tube") that is greater than a minimum separation between the shaft and a portion of the sheath that surrounds the shaft. The term "minimum separation," as it is used herein and in the claims, refers to the smallest radial distance between the sheath and the burr, in the former instance, and between the sheath and the shaft, in the later instance, at any longitudinal position along the length of the shaft and the burr. The space between the outside diameter of the shaft and the inside diameter of the sheath creates a channel along at least of portion of the sheath through which fluid and/or debris can flow.

The burr may be smaller in diameter than the shaft, while the sheath diameter may be at least slightly larger than the diameter of the shaft and approximately constant along its length. Upon lateral deflection of the shaft relative to the sheath, such as when the shaft is subject to a load created when the burr is being used to cut or abrade hard or dense tissue or bone, contact between the burr and the sheath will be avoided because the relative lateral movement of the shaft (and, thus, the burr) will be arrested by contact between the outer surface of the shaft and the inner surface of the sheath, which will occur before the burr touches the sheath.

In alternative embodiments, the diameter of the burr can be approximately the same as the diameter of the shaft, or even larger than the diameter of the shaft, and the inner diameter of the sheath can.be at least slightly larger than the diameter of the shaft in a region proximal to the burr (i.e., in a region surrounding the shaft), but flared outwards in the region surrounding the burr in an amount sufficient to provide the desired relative clearance with the burr. As with the above-described embodiment, relative lateral movement of the shaft will be arrested by the sheath prior to contact between the burr and the sheath. In some embodiments, the flaring may be a characteristic of the inner diameter of the sheath only, i.e., the outer diameter of the sheath may be substantially constant while the inner diameter of the sheath may increase in the area of the burr. The substantially uniform outside diameter provided by such an arrangement may facilitate easier insertion and removal of the shaft from an incision. Of course, the outer diameter of the sheath need not be constant and may instead have any suitable shape or contour. The precise shape of the flare of the internal/external diameter of the sheath in the region of the burr may also be tailored to a particular burr shape, so as to, for example, provide as low a profile as practicable or maintain a substantially uniform cross-sectional area of any channel formed between the shaft and the sheath.

Because contact between the burr and the sheath may be avoided in the above constructions without use of a bearing positioned in the distal region of the shaft, construction and manufacture of a rotating surgical instrument according to the invention can potentially be simplified over that of typical prior art instruments.

In some embodiments, provision of the relative minimum separations described above may be acheived by providing one or more "stand-offs" positioned in the space between the shaft and the sheath. The term "stand-off," as used herein and in the claims, refers to any structure that allows the shaft to float freely within the sheath (i.e., without direct or indirect contact between the shaft and the sheath) but is capable of maintaining a fixed distance between the shaft and the sheath upon the application of lateral force, while still allowing the shaft to be displaced laterally upon the application of lateral force. Suitable structures include, for example, ribs, feet, fins, rings, pins, knobs, ridges, buttons, or any other appropriate element or arrangement. Stand-offs may be formed integrally with the shaft and/or the sheath, may be separate structures affixed to the shaft and/or the sheath by any suitable means, or may simply be positioned in the space between the shaft and the sheath. In some embodiments, stand-offs may be formed or positioned on both the sheath and the shaft and directly adjacent to one another, such that the minimum separation lies between the stand-offs.

It should be understood that stand-offs are an optional feature and that, accordingly, many embodiments are entirely free of stand-offs. Indeed, in at least one embodiment, it is an advantageous feature that the region of the sheath and shaft distal of the body is devoid of stand-offs or any other element that might impede the flow of fluid though the sheath or that might complicate the construction and/or manufacture of the instrument.

The surgical instrument can be provided with a flexible sheath that surrounds at least a portion of a rotating shaft and, optionally, a portion of the burr as well. During operation of such instrument, the shaft may contact the sheath, but because the sheath is flexible and displacable in response to the contact, rotation of the shaft is subject to less frictional resistance than would be the case with a relatively rigid sheath. "Flexible" is used herein and in the claims to mean that the sheath itself and/or the connection between the sheath and a body of the instrument is compliant and can swivel or bend to allow the sheath to comply, at least partially, with bending or lateral displacement experienced by the shaft.

In some embodiments, the sheath (e.g., a sheath formed of a substantially rigid material) may be rendered "flexible" by reason of being affixed to the body by a connection herein referred to as a "flexible connection." A variety of types of flexible connections are contemplated, including, without limitation, types with elastomeric, resilient, and/or reversibly bendable elements, types with a swivelable and/or pivotable joint, types in which the fit between the sheath and a projecting hollow member has a large clearance, and/or even hinge-type connectors. The flexible connection between the sheath and the body in some embodiments could also be formed by corrugated or non-corrugated sections of tubing, made of essentially any resilient, medically-compatible material, linking the sheath and the body. In other embodiments, the sheath could be loosely connected to the body by a mechanical arrangement, such as a ball and socket joint or similar device, that would allow bending and/or pivoting without requiring elastic elements. Any other means for providing attachment of a sheath (e.g., one formed of a substantially rigid tube) to a body, or more particularly to a body of a surgical instrument, in a way that allows bending or pivoting of the sheath with respect to the axis of the body during operation of the instrument may also be used.

In one embodiment, the flexible connection takes the form of a boot formed of a resilient material, such as rubber or soft plastic, that fits into an annular recess in the distal end of the body. The boot can have a central bore that surrounds the shaft and a portion of the sheath and an annular recess in the bore that accepts a flared proximal end of the sheath. The flexibility of the material that forms the boot can allow the sheath to flex upon application of force with a component in a direction perpendicular to the axis of the shaft. This is but one arrangement, however, and, as described above, the invention contemplates the use of any other suitable type of flexible connection.

Instead of, or in addition to, having a flexible connection between the sheath and the body of the handpiece, some embodiments of the invention may alternatively provide a sheath at least of portion of which is flexible by reason of being composed of a resilient, supple, or pliable material and that has dimensions (e.g., thickness, diameter, etc.) that allow it to flex or bend along its length in response to the application of a lateral force. Such bending or flexing can result in less resistance to the bending and/or lateral displacement of the shaft. The flexibility of the sheath may be substantially uniform or non-uniform along its length; for example, the sheath may be more flexible in its proximal portions, so as to facilitate bending, while being less flexible in its distal portions, so as to resist external forces that might operate to push the sheath in the direction of the burr. The non-uniform flexibility may, in some cases, result from the use of a sheath that is substantially rigid but connected to the body via a flexible connector.

In one embodiment, the surgical instrument is constructed such that the sheath is more flexible than the shaft, meaning that the level of force required at a given longitudinal point to deflect the sheath a given distance is less than the level of force required at the same longitudinal point to deflect the shaft the same distance. In one embodiment, the level of force at a given longitudinal point required to deflect the sheath a given distance is less than about 90% of that required to deflect the shaft the same amount. In other embodiments, the level of force required to deflect the sheath may be less than about 75%, 50%, 25%, 10%, 5%, 2% or 1% of the force required to deflect the shaft.

It should be noted that, regardless of the means by which the sheath is made flexible, the displacement along the entire length of the sheath during operation will typically be small, in the range often degrees or less, and, more typically, a few degrees or less.

In some embodiments, a combination of the above-described features is provided; that is, the sheath has a greater clearance from the burr than it does from the shaft, and the sheath is flexible and/or is flexibly connected to the body. Such an embodiment is well suited to prevent contact between the sheath and the burr, as the clearance and the flexing of the shaft and/or the flexible connection cooperate to help maintain a separation between the sheath and the burr.

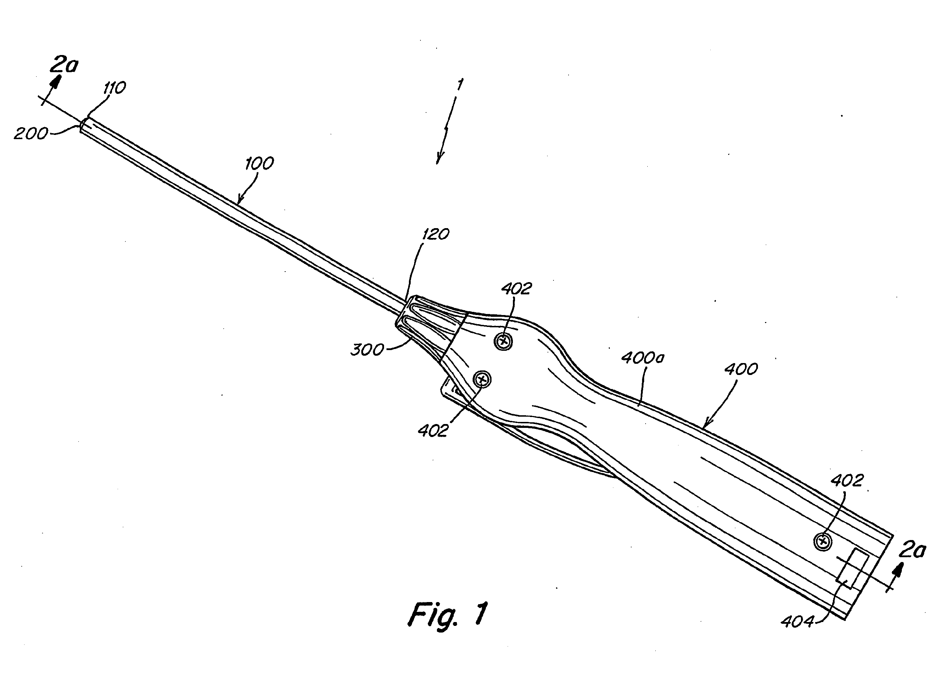

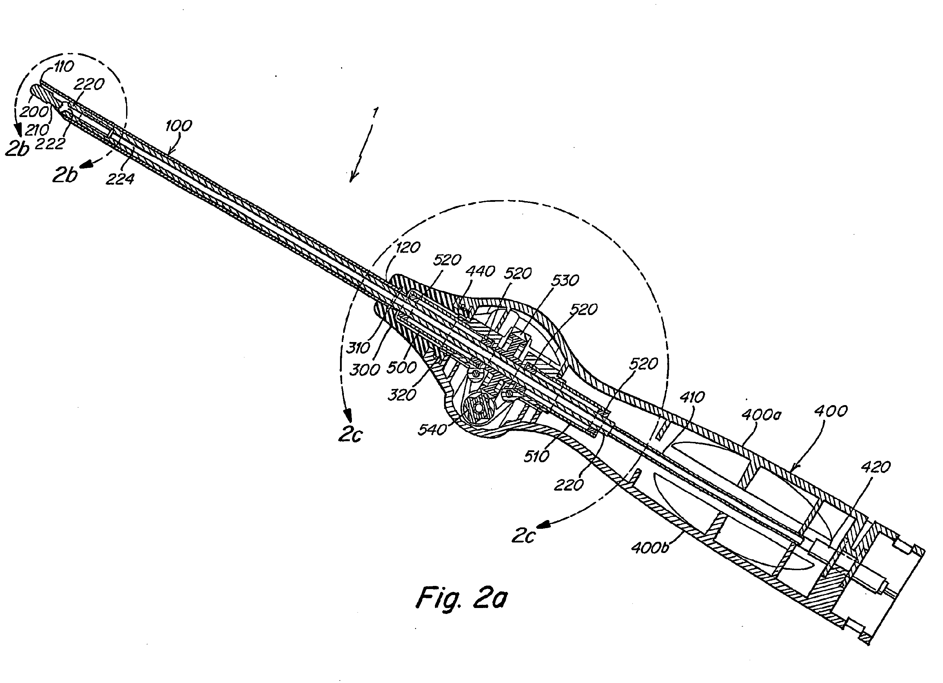

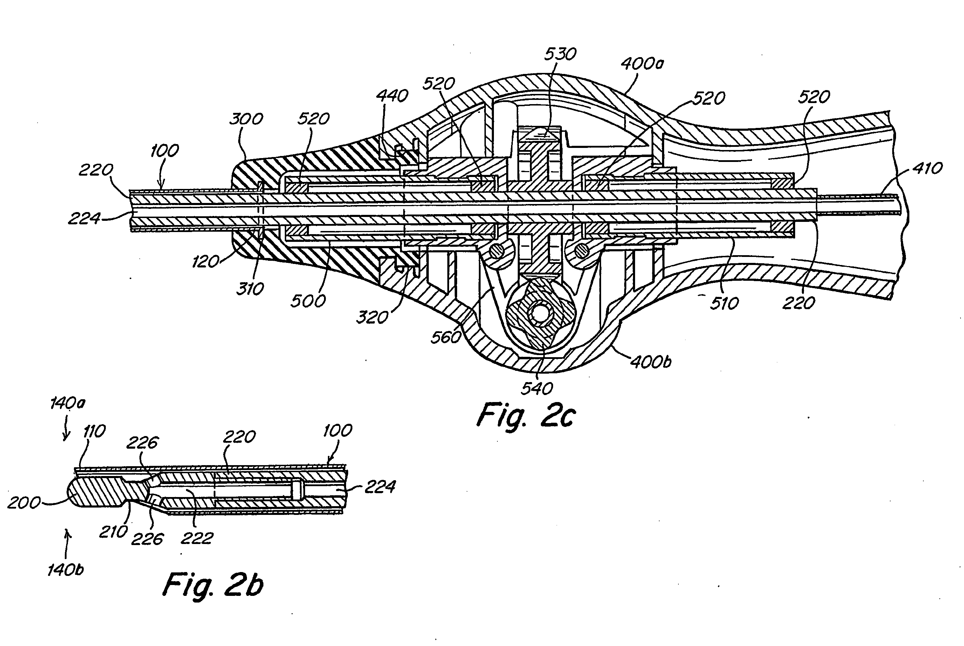

An embodiment of the invention is now described with reference to the figures to aid the experienced person in understanding certain aspects of the invention and in envisaging how it may be practiced. Figures 1 and 2 show one exemplary surgical instrument according to the invention. As shown in Fig. 1, the exemplary device 1 includes a sheath **100** with a distal tip **110** and a proximal end **120,** a shaft **220** (see Fig. 2a), and a burr **200.**

Figure 2a, and enlargements 2b and 2c, show cross-sectional views of the surgical instrument of Fig. 1. Fig. 2b shows an enlarged view of the burr **200,** shaft **220,** and sheath **100.** The burr **200** of this embodiment is manufactured in a single piece with neck **210** and shaft **220.** In other embodiments, however, the burr **200,** neck **210,** and shaft **220** may be assembled after manufacture, either reversibly or irreversibly. Suitable methods of joining these components include the use of a tang that is optionally hidden after the connection is made, direct welding, threads, a press fit, or any other appropriate method for joining cylindrical objects end-to-end. In other embodiments, the neck **210** may be diminished, or omitted entirely, so that the burr **200** is directly connected to the shaft **220.** The burr **200** of this embodiment has a diameter that is smaller than that of the shaft **220.** As noted above, however, in other embodiments, the burr **200** may be of the same or a larger diameter than the shaft **220.**

While this embodiment includes a burr **200** with a shape particularly suitable for abrading tissue, it should be understood that any type of abrasive, cutting, polishing, or other tissue contacting element is potentially suitable for use in place of the burr **200** according to the invention. The teeth of the burr **200,** for instruments including burrs with teeth, may be cut into a body, as is conventional, or may be provided in the form of raised ridges of various profiles. Such profiles can be effective, even for fairly smooth profiles, due to the high rotational speeds achievable by some embodiments of instruments provided according to the invention. In some embodiments, a burr may be provided that comprises lateral linear projections from a body portion, such as wires or polymeric bristles, which may be flexible or rigid, so that the burr can act as a brush that, for example, can scour tissue to which it is applied. In other, related embodiments, a burr may be utilized that is analogous in structure and function to a rotary string trimmer/cutter. In some such embodiments, the burr can comprise one or more components including a feed mechanism for supplying one or more cutting filaments formed, for example, of polymer, metal wire, etc., in a cutting/trimming configuration. Such feed mechanism can, in some such embodiments, be configured to reversibly feed such filaments through apertures in the feed mechanism, such that abrasion, trimming, and/or cutting can be achieved upon rotation of a shaft of the instrument to which such feed mechanism is attached. In certain of such embodiments, the feed mechanism can comprise a head, which includes the apertures and contains the filaments, carried on the shaft.

The burr **200** may be made of a material that can keep a sharp edge and that will not react chemically with the tissue. Metals, including steel, more especially stainless steel, are most commonly used for such purposes, and often the steel or other metal will be hardened. Ceramic cutters, or cutters coated with hard ceramic particles, diamond dust, other abrasives, or grit are also known. In very high speed burrs, some plastics may provide sufficient cutting action for soft tissue, or may be suitable for polishing.

The shaft **220** of this embodiment is approximately cylindrical has a central lumen **224.** In other embodiments, however, the shaft **220** may be wholly or partially solid. The length and diameter of the shaft **220** may vary depending on the application although, in certain embodiments, the diameter is approximately constant along its length. The shaft **220** can be made of stainless steel, but, alternatively, it may be made of other materials having sufficient strength, including, without limitation, metals, and particularly metals selected from other steels, aluminum, titanium and bronze. Those of ordinary skill in the art will, based on well-known material property data and no more than routine experimentation, readily be able to evaluate candidate materials or combinations of materials and determine if their properties are appropriate.

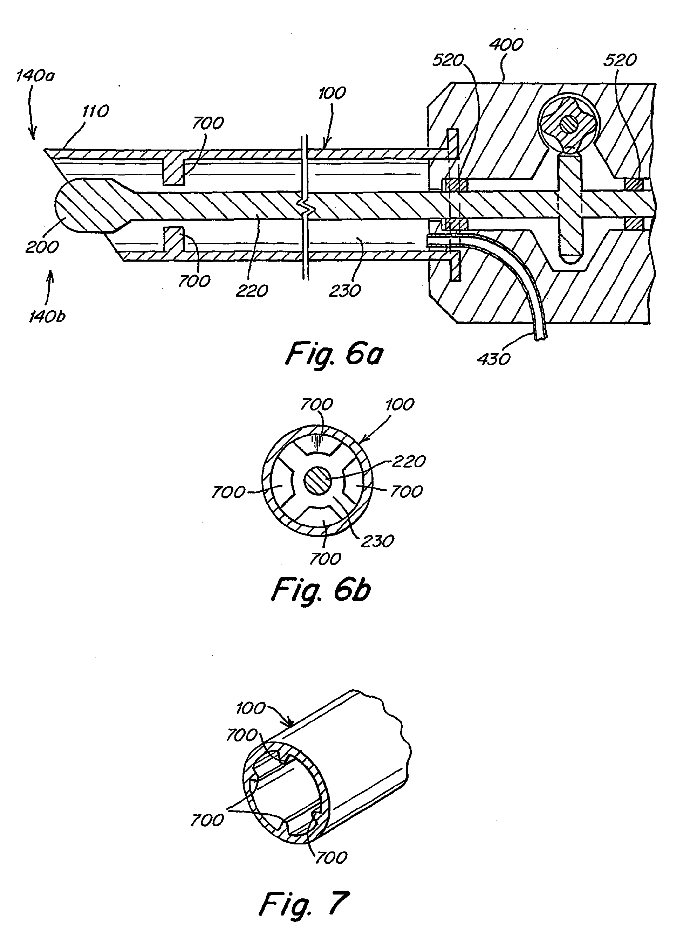

In the illustrated embodiment, the shaft **220** and burr **200** are both at least partially surrounded by sheath **100.** There is separation between the shaft **220** and the sheath **100** which forms a channel **230** (shown in Fig. 6a). In this embodiment, the minimum separation between the burr **200** and sheath **100** is greater than the minimum separation between the shaft **220** and the sheath **100.** As a result of the difference in these minimum separations, upon the application of a lateral force to the burr **200,** the shaft **220** may deflect relative to the sheath **100** until the shaft contacts the sheath **100.** Once the shaft **220** contacts the sheath **100,** the relative deflection will stop and the burr **200** will be prevented from contacting the sheath **100.**

In some alternative embodiments (not shown), the distance between the sheath and the shaft may not be constant along the length of the shaft. For example, the inner diameter of the sheath may be larger than the outside diameter of the shaft by a certain amount for some distance from the proximal end of the shaft and then may have a region in which the difference in the diameters is smaller, forming a neck in a mid-region of the shaft that is the location of the minimum separation, and then may widen in the region distal and/or proximal to the neck.

In the illustrated embodiments, the sheath **100** is configured as a tube, which may be made of a material that is more easily displaced laterally than the shaft, but that has a sufficient stiffness to resist significant bending deformation by the lateral stresses likely to be encountered in ordinary use. In particular, the sheath **100** may be constructed to be rigid enough to prevent it from coming into contact with the burr **200,** while being sufficiently movable with respect to the body that it can, under expected operating conditions, flex, bend, or pivot along its length in response to the application of a lateral force. The bending, flexing, or pivoting may result in less resistance to the bending of the shaft **220.** As noted above, the flexibility of the sheath **100** can also vary, in some embodiments, along its length, and such variable flexibility may be effected, for example, by varying the composition of the material that forms the sheath **100,** by varying the thickness of all or part of the sheath **100,** or by the inclusion of a flexible connector.

The sheath **100** of some embodiments of the invention, such as that illustrated in Figs. 1 and 2, may also function to prevent contact between the shaft **220** and adjacent tissue. Some such embodiments may provide a sheath **100** configured such that the sheath covers all or part of the shaft **220,** an arrangement that can prevent damage to adjacent tissue that might result from friction or from imperfections, such as scratches, on the shaft **220.** In some embodiments, and as illustrated in Figs. 1 and 2, the sheath **100** may fully encircle the shaft, so as to protect tissue from contact with the rotating shaft **220** around its entire circumference. In other embodiments, the sheath may cover only a portion of the shaft, leaving other portions of the shaft exposed. In still further embodiments, the sheath may have any of a variety of perforations, in the form of holes, slots, slits, or the like to, for example, reduce the weight of the instrument or to facilitate various fluid flow arrangements, while still providing sufficient strength and/or shielding.

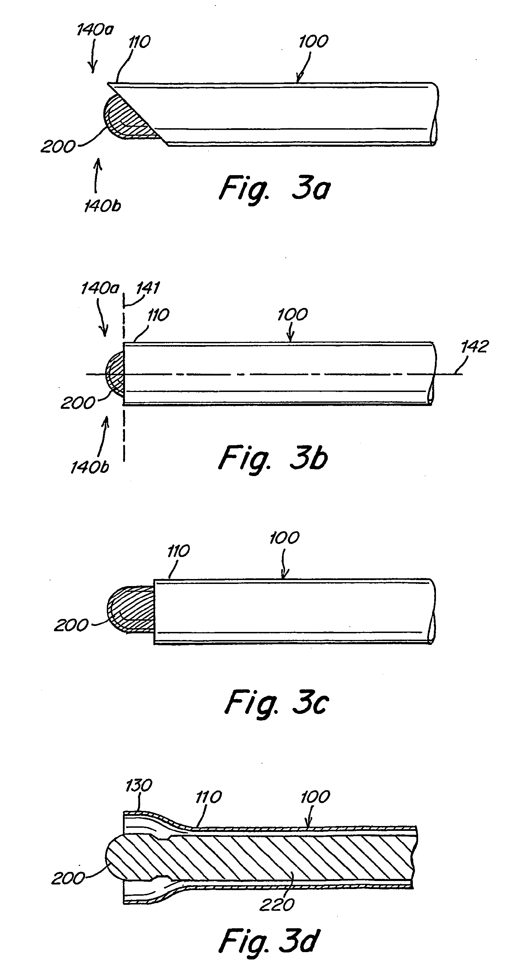

In certain embodiments, the sheath may surround only a portion, or even variable portions, of the burr. In some embodiments, for example, the sheath may cover only a portion of the burr. Such an arrangement is shown in Fig. 2b, where the sheath **100** extends nearly to the distal end of the burr **200** on the top side **140a** of the distal end **110,** but is cut away, such that the distal end of the sheath **100** forms an acute angle with the longitudinal axis of the shaft **220,** to reveal a large portion of the burr **200** on the bottom side **140b** of the distal end **110.** This type of construction provides protection to tissues located on the top side **140a,** while allowing the burr **200** to contact tissue located on the bottom side 140b. A non-sectional view of a similar arrangement is shown in Fig. 3a. Alternatively, the distal end **110** of the sheath **100** may terminate in a plane **141** perpendicular to the longitudinal axis **142** of the shaft **220** and passing through the burr **200,** as shown in Fig. 3b, in which case a larger or smaller portion of the burr **200** may be exposed, depending on where the distal end of the sheath **100** stops in relation to the burr **200.** The sheath 100 may also be constructed and positioned to expose substantially all of the burr **200,** as shown in Fig. 3c.

In the embodiment illustrated in cross-section in Fig. 3d, a portion of the sheath 100 is expanded in diameter at its distal end to form, for example, a hood **130,** so as to increase the separation between the inner surface of the hood **130** and the burr **200.** The sheath **100** may also have any of a number of other configurations, as would be apparent to one of skill in the art, that would expose only selected portions of the burr **200.**

In still other embodiments, the sheath may be constructed and arranged so that the point at which the distal end of the sheath terminates may be variable, allowing the amount of the burr that is exposed to be changed. Such variable exposure may be effected, for example, by a bellows-type arrangement, a slidable or threaded sheath, a telescoping sheath arrangement, or any other suitable method, as would be appreciated by those of skill in the art. In some such embodiments, the exposure of the burr may be adjusted during the course of a procedure, either manually or by some type of automatic control.

In general, the sheath **100** may have any suitable thickness and external diameter and may be constructed of any appropriate material, selection of which dimensions and materials is well within the abilities of one of skill in the art given the guidance and teaching of the present description. In one exemplary embodiment, the sheath **100** is made of stainless steel, but, alternatively, it may be made of other materials having sufficient strength, including without limitation metals, and particularly metals selected from other steels, aluminum, titanium, bronze, and copper and its alloys. In some embodiments, the sheath **100** may be made of rigid plastic, although the material should preferably be non-melting under expected operating conditions. In some embodiments, the sheath **100** may be optically transparent to aid visualization of the burr **200** and/or may be provided with a radiopaque portion or element to further facilitate visualization. In still other embodiments, the sheath **100** may be made of a combination of one or more of these materials. In still other embodiments, the sheath may be made of a substantially resilient, pliant, and/or flexible material, as previously discussed. As with the shaft **200,** those of ordinary skill in the art will, based on well-known material property data and routine experimentation, readily be able to evaluate candidate materials to see if they possess appropriate properties. The material of the sheath **100** may be selected in certain embodiments from a material that does not readily gall on contact with the burr **200.**

For embodiments in which removal of fluid or debris from the surgical site is desired, it can be effected by providing for removal through a lumen in the shaft and/or by providing a channel between the shaft and the sheath. In embodiments in which the shaft has a lumen, the shaft may be provided with openings, which may, in some embodiments, be positioned near its distal end, to allow fluid and/or debris to flow into the lumen. The embodiment of Figs. 1 and 2, for example, includes a hollow shaft **220** providing a lumen **224** with inlets **226** that fluidly connect the area surrounding the burr **200** with the entry lumen **222.** When used, inlets **226** may, in certain embodiments, be arranged symmetrically around the shaft **220,** for balance during rotation. As shown in Fig. 2c, the entry lumen **222** is fluidly connected to shaft lumen **224,** and shaft lumen **224** is in fluid communication with a proximal tube **410** that extends from the proximal end of the shaft **220** and passes through a carrier block **420** (see Fig. 2a) to the proximal end of the body. Providing for the evacuation of fluid through a channel **230** between the shaft **220** and the sheath **100** is particularly suitable for embodiments of the invention in which the region of the sheath **100** and shaft **220** distal of the body is free of any support member or other element tending to obstruct channel **230.** In typical embodiments, the suction necessary to induce and maintain evacuation flow through the lumen **224** and/or the channel **230** can be provided, for example, by elevation of a bag of saline used for irrigating the surgical site or by the design of the burr to act as an impeller as it rotates. In some embodiments, it is contemplated that fluid could be delivered to or evacuated from the site through either or both of a shaft lumen or a channel between the shaft and the sheath, either simultaneous or sequentially.

Some embodiments may also include a flexible connector positioned between the sheath and the body. In the embodiment shown in Fig. 2c, for example, the sheath **100** passes into the connector **300** and the flared proximal end **120** of the sheath **100** is held in an annular groove **310.** The connector **300** of this embodiment is made of a flexible, resilient material, such as a rubber. In other embodiments, the connector **300** may take other shapes than illustrated and/or may be made of other flexible materials, such as, for example, other resilient polymeric materials and certain metals. As illustrated, connector **300** has a rim **320** that fits into a slot **440** in body **400.** Because the connector **300** is flexible, application of lateral force to the sheath **100** or the burr **200,** for example via contact with the shaft **220** during operation, will tend to cause the connector **300** to flex in the direction of the applied force, which will cause the sheath **100** to pivot in that direction with respect to the flexible connector **300,** thereby reducing the possibility that the burr **200** might contact the sheath **100** and reducing the friction between the shaft **220** and the sheath **100.** In alternative embodiments, the rubber flexible connector **300** may be replaced by, and/or supplemented with, a connector made of other types of elastomeric, resilient, and/or reversibly bendable materials. In other alternative embodiments, the illustrated flexible connector may be replaced by and/or supplemented with a pivotable mechanical connection, such as a swivelable and/or pivotable joint, a hinge, corrugated sections of tubing, a ball and socket joint or other similar device, that would allow bending and/or pivoting with or without requiring elastic elements. In essence, essentially any suitable means for providing attachment of a tube to a body of a surgical instrument in a way that would allow relatively easy bending or pivoting of the tube with respect to the axis of the body can potentially be employed within the scope of the invention.

Surgical device 1 illustrates an embodiment that includes an optional body **400.** While the body **400** of the illustrated embodiment is formed of two injection molded plastic sides **400a, 400b,** in other embodiments the body **400** may be formed of any material suitable for use in a surgical instrument including, without limitation, any appropriate plastic or metal and may be formed of any suitable number of parts, including one. Where the body **400** is formed from more than one interconnected piece, the pieces may be held together by any suitable means. In the embodiment of Figs. 1 and 2, for example, screws **402** are illustrated. In other embodiments, however, the screws **402** could be replaced by rivets, clamps, adhesives, a snap fit, or any other appropriate method of fastening the parts of the body together. The exterior of the body **400** may also be sized and shaped to be held in a hand, as shown, and may be adapted to reversibly or irreversibly mate with a device (not shown) that provides feed lines for fluid influx and efflux. In the illustrated embodiment, a locking slot **404** at the distal end of the body accommodates a tab (also not shown) for attaching the body **400** to such a device.

In some embodiments, as illustrated in Figs. 1 and 2, the body **400** also houses support members configured and positioned for rotatably supporting the shaft. Such support members may take the form of, for example, roller or non-roller bearings, bushings, o-rings, washers, ribs, feet, fins, rings, pins, knobs, ridges, buttons, or any other appropriate element or arrangement, as would be apparent to one of skill in the art. Within the body **400** of the illustrated embodiment, for example, a first support tube **500** and a second support tube **510** each carry two support members **520** that rotatably support the shaft **220.** The support members **520** may fully encircle the shaft **220** or may be discontinuous around the circumference of the shaft, so long as they are constructed positioned so as to provide appropriate rotational support. In some embodiments, the support members **520** may simply be portions (e.g. molded portions) of the body of the body itself which are constructed and arranged to rotatably support the shaft.

In the embodiment of Figs. 1 and 2, the shaft **220** is rotatably supported solely by support members **520,** each of which are positioned within the body **400,** thus resulting in a cantilevered arrangement. Such an arrangement can provide sufficient lateral support of the shaft **220** to allow the device to be used on hard tissue without the burr **200** contacting the sheath, particularly when used in conjunction with the minimum separation, flexible sheath, and/or flexible connector arrangements described above for preventing burr-shaft contact. This type of support member arrangement can also facilitate a construction, such as that illustrated, in which the region of the sheath **100** and shaft **220** distal of the body **400** is devoid of any bearings, support members, or elements that might block the flow of fluid though the space between the sheath **100** and shaft **220** and/or complicate the construction and/or manufacture of the instrument. In other embodiments other than those illustrated, the support members may be positioned wholly or partially outside of the body, on either its distal or its proximal side.

As illustrated in Figs. 1 and 2, the shaft **220** may be drivingly connected and/or affixed to a driving element (also referred to herein and in the claims as a "motor"), which may be any device or arrangement capable of imparting rotation to the shaft. The motor of the illustrated embodiment is a liquid-jet driven rotor drive mechanism similar to that described in commonly owned co-pending U.S. Patent Application Serial No. 09/480,500 and International Publication No. WO 01/50966.

This drive mechanism can deliver both high speed and high torque, and tends to slow and stall smoothly as torque increases. In alternative embodiments, however, other drive mechanisms may be utilized in the invention. In particular, an air turbine is may be used in certain embodiments, as may an electric motor.

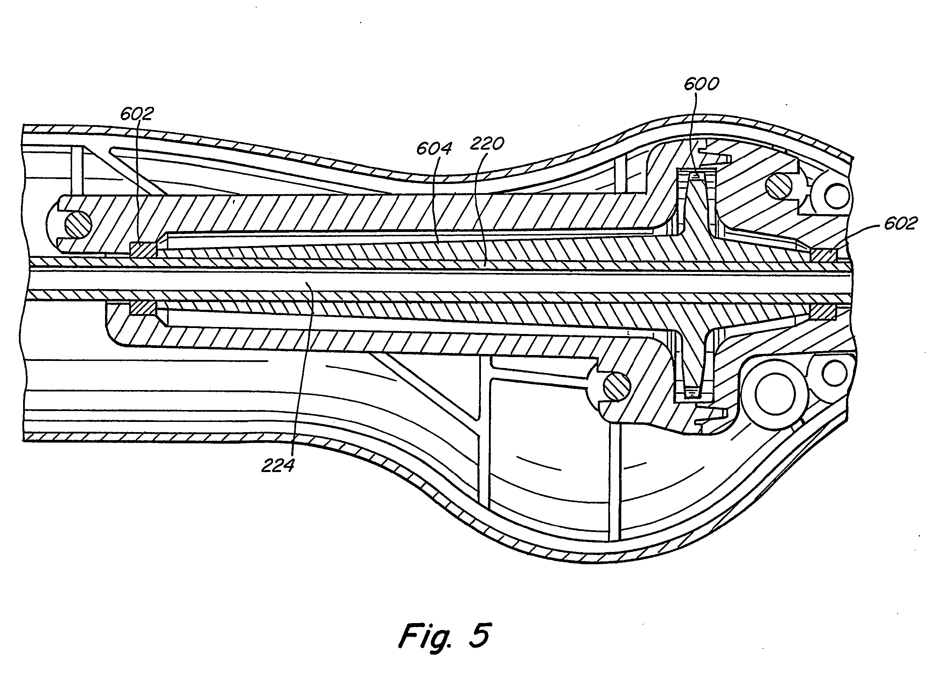

In the embodiment illustrated in Figs. 1, 2, and 4, a driving gear **530** connects the shaft **220** to the liquid jet-driven rotor **550.** The gear **530** can be made of any suitable material, including but not limited to metal and plastic, may be any suitable shape, and may be affixed to the shaft by any suitable means, as would be apparent to those skilled in the art. In other embodiments, the shaft **220** and the gear **530** may be formed from a single piece of material. Figure 5, for example, depicts an alternative embodiment in which the water-jet driven rotor **600** is coupled directly to the shaft **220.** The shaft **220** is supported by two support members **602,** and encased by a support member **604,** which combines the functions of the first **500** and second **510** support tubes, and the connecting block **12** of the previous embodiment, illustrated in Figs. 1, 2, and 4.

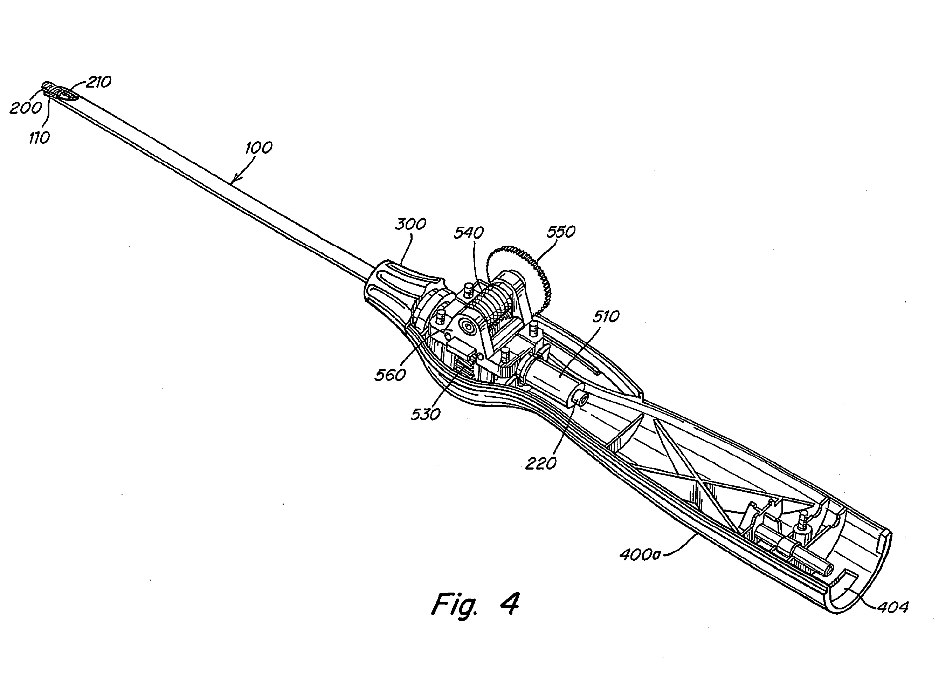

Referring again to Fig. 4, the gear **530** of this embodiment is driven by a worm gear **540** that is, in turn, driven by a rotor **550,** and all of these components are held by a connector block **560,** which also holds distal and proximal support tubes **500, 510.** The connector block **560** is attached to the body **400.** In operation, the rotor **550** drives the worm gear **540** which, in turn, drives the gear **530.** The gear **530** rotates the shaft **220,** and the shaft **220** rotates the burr **200.**

In various embodiments, the connection between any motor and the shaft **220** may be reversible and/or may also be indirect, such as, for example, through a belt, a shaft, a hose, or one or more gears. In some embodiments, the motor may be partially or wholly external to the body **400** as, for example, where the motor is a fluid-driven motor in fluid communication with a source of pressurized fluid delivered to a turbine or rotor within the body or is an externally positioned electric motor drivingly coupled via a flexible drive shaft or other suitable means to the shaft of the instrument. In some alternative embodiments (not shown), the rotatable shaft may be inserted into a collet, a chuck, or a similar device, which is itself directly or indirectly driven by a motor. The collet, chuck, or similar device may be mounted within the body or may be on the outside of the body. In still other embodiments, rotation of the shaft and, in turn, the burr, may be effected without a motor, for example, by hand.

Burr rotation speeds achievable by instruments provided according to certain embodiments of the invention are not limited, but may be at least 5,000 rpm, at least about 10,000 rpm, or at least about 20,000 rpm. With suitable motors (e.g., certain liquid-jet driven rotor motors), speeds of at least 30,000 rpm, or at least 50,000 rpm, or of over 100,000 rpm, can be obtained with some embodiments of the invention.

Figure 6a shows an embodiment of the invention that employs stand-offs. The burr **200,** which in the illustrated embodiment has a diameter larger than that of the shaft **220,** is mounted on the shaft **220,** which may be either solid (as illustrated) or hollow, as described above. The shaft **220** is driven by a motor, such as a turbine, a liquid-jet driven rotor motors, or an electric motor, that is located in the body **400.** The shaft **220** is supported by two support members **520** that are also located in the body **400.** As in other embodiments, the shaft **220** is surrounded by a sheath **100.** The sheath **100** of this embodiment extends to the end of the burr **200** on the top side **140a,** but is tapered so as to reveal a portion of the burr **200** on the bottom side **140b.**

The inner surface of the sheath **100** of this embodiment is provided with stand-offs **700,** which may be of any suitable design and are constructed to tolerate intermittent contact with the rotating shaft **220** during operation upon application of sufficient lateral force to the burr **200** and shaft **220.** The burr **200** has a minimum separation from the surrounding sheath **100** that is greater than the minimum separation between the stand-offs **700** and the shaft **220.** As such, upon lateral deflection of the shaft **220** relative to the sheath **100,** contact of the burr **200** with the sheath **100** will be avoided because the relative lateral movement will be arrested when the minimum separation at the stand-offs **700** drops to zero. This will occur before the burr **200** contacts the sheath **100.**

Stand-offs **700** do not contact both the sheath **100** and the shaft **200** during normal operation of the instrument; rather, in the absence of lateral forces, there is a minimum separation between the shaft **220** and the stand-offs **700** (where the stand-off **700** is adjacent to or a part of the sheath **100,** as illustrated), between the sheath 100 and the stand-off **700** (where the stand-off **700** is adjacent to or a part of the shaft **220),** or, in alternative embodiments (not shown), between two stand-offs **700** (where one stand-off **700** is adjacent to or a part of the shaft **220** and one stand-off **700** is adjacent to or part of the sheath **100).** Stand-offs **700** can, in some embodiments, be shaped and arranged so that the amount of deflection of the shaft **220** possible in all radial directions is approximately the same (and, in any event, is less in all directions than the amount of deflection sufficient for the burr **200** to contact the sheath **100).** This may be accomplished, in certain embodiments, by the use of one or more annular stand-offs (e.g., annular ribs) or by the use of individual stand-offs that may be discrete structures uniformly spaced around the inside of the sheath, or, alternatively, by the use of longitudinal ribs, as shown in Fig. 7.

Referring again to Fig. 6, stand-offs **700** may be positioned at any suitable point along the length of the shaft **220** and, in some embodiments, more than one stand-off, of the same or different types, may be used. In some embodiments, it may be advantageous to position the stand-off(s) **700** in a distal region of the shaft, because, due bending along the length of the shaft **220,** stand-off(s) **700** at such a position may be more effective at arresting relative deflection of the shaft **220** than would be a stand-off(s) at a more proximal position. In other words, because the displacement of a bending shaft relative to its original axis may be greater at its distal end than at its proximal end, stand-off(s) positioned near the distal end of the shaft **220** may contact the bending shaft **220** earlier than would the same stand-off(s) at a more proximal position. In one embodiment, as illustrated, stand-offs **700** are positioned at a single longitudinal position just proximal of the burr **200.**

In some embodiments employing stand-offs, it may be desirable to facilitate the flow of fluid through the sheath **220** in the space between the shaft **220** and the sheath **100.** In such cases, stand-offs in the fonn of distinct knobs, pins, longitudinal ribs, or the like are advantageously used, or, alternatively, annular stand-offs that have one or more openings (holes, slots, notches, etc.) may be employed, to allow for improved fluid flow in the axial direction as compared to annular stand-off(s) without such openings. In the embodiment illustrated in Figure 6b, for example, the stand-offs **700** comprise discontinuous annular ribs that are configured to allow fluid and debris to flow from the area of the burr **200,** through the channel **230** formed between the shaft **220** and the sheath **100,** and through the tube **410.** Because the debris is generally small in diameter compared to the channel **230,** only a small amount of suction is typically required to remove the debris from the area of the burr **200.** Sufficient suction can be provided, in some embodiments, by elevation of a bag of saline (not shown) used for irrigating the surgical site. The flow of fluid away from the surgical site may also be facilitated by the design of the burr **200,** as previously described.

In some embodiments, the outer surface of the shaft **220,** the inner surface of the sheath **100,** and/or various surfaces of the stand-offs **700** are provided with a relatively smooth finish, so as to prevent damage upon any of these surfaces coming into contact with each other. In some embodiments, these surfaces may covered with a protective and/or lubricious coating to minimize friction.

## Claims

1. A rotatable component-providing surgical instrument, comprising:
a body (400) having a distal end and a proximal end;
a shaft (220) rotatably supported by the body and extending from the distal end of the body in a cantilevered arrangement, without use of a bearing in the distal region of the shaft;
a tissue-contacting component (200) drivable by the shaft; and
an outer tube (100) connected to the distal end of the body and surrounding at least a portion of the shaft, wherein a minimum separation between the tissue-contacting component and the outer tube is greater than a minimum separation between the shaft and the outer tube.

2. The surgical instrument of claim 1, wherein the shaft has a longitudinal axis and the outer tube is constructed and arranged to be movable in the direction of the axis.

3. The surgical instrument of claim 1, wherein the outer tube further comprises a radiopaque marker.

4. The surgical instrument of claim 1, wherein the shaft further comprises a lumen (222) adapted for fluid flow therein.

5. The surgical instrument of claim 1, wherein the outer tube is flexible.

6. The surgical instrument of claim 1, further comprising an evacuation channel (230).within the outer tube.

7. The surgical instrument of claim 1, further comprising an irrigation channel (230) within the outer tube.

8. The surgical instrument of any one of the preceding claims, wherein the outer tube is constructed and arranged such that, upon application of lateral force to the tissue-contacting component, the outer tube contacts the shaft before contacting the tissue-contacting component.

9. The surgical instrument of any one of the preceding claims, further comprising:
a flexible connector (300) member positioned on the distal end of the body flexibly connecting the outer tube to the distal end of the body, thereby rendering the outer tube more flexible than the shaft with respect to the body.

10. The surgical instrument of claim 9, further comprising at least one stand-off (700) positioned between the shaft and the outer tube.

11. The surgical instrument of claim 1, further comprising a flexible connector positioned at least in part between the body and the outer tube.

12. Instrument as claimed in claim 1, in which the outer tube is more flexible than the shaft with respect to the body.

13. The surgical instrument of claim 1, wherein the resistance to deflection of the outer tube to a laterally applied force at a given longitudinal point is at least 10 percent less than the resistance to deflection of the shaft to the same force at the same longitudinal point.

14. The surgical instrument of claim 13, wherein the resistance to deflection of the outer tube to a laterally applied force at a given longitudinal point is at least 50 percent less than the resistance to deflection of the shaft to the same force at the same longitudinal point.

15. The surgical instrument of claim 14, wherein the resistance to deflection of the outer tube to a laterally applied force at a given longitudinal point is at least 90 percent less than the resistance to deflection of the shaft to the same force at the same longitudinal point.

16. The surgical instrument of any one of the preceding claims, wherein the flexibility of the outer tube at a first longitudinal point is different than the flexibility of the outer tube at a second longitudinal point.

17. The surgical instrument of any one of the preceding claims, wherein a maximum radial diameter of the tissue-contacting component is smaller than a minimum radial diameter of the shaft.

18. The surgical instrument of any one of claims 1 to 17, wherein a maximum radial diameter of the tissue-contacting component is approximately the same as a maximum radial diameter of the shaft.

19. The surgical instrument of any one of the preceding claims, wherein an interior diameter of the outer tube (100) in a region adjacent to the shaft is smaller than an interior diameter of the outer tube in a region adjacent to the tissue-contacting component.

20. The surgical instrument of claim 11, wherein the flexible connector is a resilient boot.

21. The surgical instrument of any one of the preceding claims, wherein the outer tube surrounds at least a portion of the tissue-contacting component.

## Patentansprüche

1. Drehbares Komponenten bereitstellendes chirurgisches Instrument, umfassend:
einen Körper (400) mit einem distalen Ende und einem proximalen Ende;
einen Schaft (220), der vom Körper drehbar getragen wird und sich aus dem distalen Ende des Körpers in einer freitragenden Anordnung erstreckt, ohne Verwendung eines Lagers im distalen Bereich des Schafts;
eine Gewebe berührende Komponente (200), die vom Schaft antreibbar ist; und
ein äußeres Rohr (100), das mit dem distalen Ende des Körpers verbunden ist und mindestens einen Teil des Schafts umgibt, wobei ein minimaler Abstand zwischen der Gewebe berührenden Komponente und dem äußeren Rohr größer als ein minimaler Abstand zwischen dem Schaft und dem äußeren Rohr ist.

2. Chirurgisches Instrument nach Anspruch 1, wobei der Schaft eine Längsachse besitzt und das äußere Rohr so aufgebaut und angeordnet ist, dass es in der Richtung der Achse beweglich ist.

3. Chirurgisches Instrument nach Anspruch 1, wobei das äußere Rohr weiter eine röntgendichte Markierung umfasst.

4. Chirurgisches Instrument nach Anspruch 1, wobei der Schaft weiter ein Lumen (222) umfasst, das für einen Fluidfluss darin geeignet ist.

5. Chirurgisches Instrument nach Anspruch 1, wobei das äußere Rohr flexibel ist.

6. Chirurgisches Instrument nach Anspruch 1, weiter einen Absaugkanal (230) im äußeren Rohr umfassend.

7. Chirurgisches Instrument nach Anspruch 1, weiter einen Spülkanal (230) im äußeren Rohr umfassend.

8. Chirurgisches Instrument nach einem der vorangehenden Ansprüche, wobei das äußere Rohr so aufgebaut und angeordnet ist, dass das äußere Rohr bei Ausüben einer seitlichen Kraft auf die Gewebe berührende Komponente den Schaft berührt, bevor es die Gewebe berührende Komponente berührt.

9. Chirurgisches Instrument nach einem der vorangehenden Ansprüche, weiter umfassend:
ein flexibles Verbinderelement (300), das am distalen Ende des Körpers positioniert ist und dabei das äußere Rohr flexibel mit dem distalen Ende des Körpers verbindet, wodurch das äußere Rohr in Bezug auf den Körper flexibler als der Schaft gemacht wird.

10. Chirurgisches Instrument nach Anspruch 9, weiter mindestens ein zwischen dem Schaft und dem äußeren Rohr positioniertes Abstandselement (700) umfassend.

11. Chirurgisches Instrument nach Anspruch 1, weiter einen mindestens teilweise zwischen dem Körper und dem äußeren Rohr positionierten flexiblen Verbinder umfassend.

12. Instrument nach Anspruch 1, bei dem das äußere Rohr in Bezug auf den Körper flexibler als der Schaft ist.

13. Chirurgisches Instrument nach Anspruch 1, wobei der Widerstand gegen eine Biegung des äußeren Rohrs bei einer seitlich ausgeübten Kraft an einem gegebenen longitudinalen Punkt mindestens 10 % geringer ist als der Widerstand gegen eine Biegung des Schafts bei derselben Kraft am selben longitudinalen Punkt.

14. Chirurgisches Instrument nach Anspruch 13, wobei der Widerstand gegen eine Biegung des äußeren Rohrs bei einer seitlich ausgeübten Kraft an einem gegebenen longitudinalen Punkt mindestens 50 % geringer ist als der Widerstand gegen eine Biegung des Schafts bei derselben Kraft am selben longitudinalen Punkt.

15. Chirurgisches Instrument nach Anspruch 14, wobei der Widerstand gegen eine Biegung des äußeren Rohrs bei einer seitlich ausgeübten Kraft an einem gegebenen longitudinalen Punkt mindestens 90 % geringer ist als der Widerstand gegen eine Biegung des Schafts bei derselben Kraft am selben longitudinalen Punkt.

16. Chirurgisches Instrument nach einem der vorangehenden Ansprüche, wobei die Flexibilität des äußeren Rohrs an einem ersten longitudinalen Punkt verschieden von der Flexibilität des äußeren Rohrs an einem zweiten longitudinalen Punkt ist.

17. Chirurgisches Instrument nach einem der vorangehenden Ansprüche, wobei ein maximaler radialer Durchmesser der Gewebe berührenden Komponente kleiner als ein minimaler radialer Durchmesser des Schafts ist.

18. Chirurgisches Instrument nach einem der Ansprüche 1 bis 17, wobei ein maximaler radialer Durchmesser der Gewebe berührenden Komponente ungefähr derselbe wie ein maximaler radialer Durchmesser des Schafts ist.

19. Chirurgisches Instrument nach einem der vorangehenden Ansprüche, wobei ein Innendurchmesser des äußeren Rohrs (100) in einem Bereich neben dem Schaft kleiner ist als ein Innendurchmesser des äußeren Rohrs in einem Bereich neben der Gewebe berührenden Komponente.

20. Chirurgisches Instrument nach Anspruch 11, wobei der flexible Verbinder eine elastische Manschette ist.

21. Chirurgisches Instrument nach einem der vorangehenden Ansprüche, wobei das äußere Rohr zumindest einen Abschnitt der Gewebe berührenden Komponente umgibt.

## Revendications

1. Instrument chirurgical rotatif fournissant un composant, comprenant :
un corps (400) comportant une extrémité distale et une extrémité proximale;
un arbre (220) supporté avec faculté de rotation par le corps et s'étendant depuis l'extrémité distale du corps dans un agencement en porte à faux, sans utiliser un roulement dans la région distale de l' arbre ;
un composant venant en contact avec du tissu (200) pouvant être entraîné par l'arbre ; et
un tube externe (100) raccordé à l'extrémité distale du corps et entourant au moins une partie de l'arbre, dans lequel une séparation minimale comprise entre le composant venant en contact avec du tissu et le tube externe est supérieure à une séparation minimale comprise entre l'arbre et le tube externe.

2. Instrument chirurgical selon la revendication 1, dans lequel l'arbre présente un axe longitudinal et le tube externe est construit et disposé pour être mobile dans le sens de l'axe.

3. Instrument chirurgical selon la revendication 1, dans lequel le tube externe comprend en outre un marqueur radio-opaque.

4. Instrument chirurgical selon la revendication 1, dans lequel l'arbre comprend en outre une lumière (222) adaptée pour un écoulement de fluide dans celle-ci.

5. Instrument chirurgical selon la revendication 1, dans lequel le tube externe est souple.

6. Instrument chirurgical selon la revendication 1, comprenant en outre un canal d'évacuation (230) à l'intérieur du tube externe.

7. Instrument chirurgical selon la revendication 1, comprenant en outre un canal d'irrigation (230) à l'intérieur du tube externe.

8. Instrument chirurgical selon l'une quelconque des revendications précédentes, dans lequel le tube externe est construit et agencé de telle sorte que, lors de l'application d'une force latérale sur le composant venant en contact avec du tissu, le tube externe vient en contact avec l'arbre avant de venir en contact avec le composant venant en contact avec du tissu.

9. Instrument chirurgical selon l'une quelconque des revendications précédentes, comprenant en outre :
un élément de raccord souple (300) positionné sur l'extrémité distale du corps, raccordant de façon souple le tube externe à l'extrémité distale du corps, rendant de ce fait le tube externe plus souple que l'arbre par rapport au corps.

10. Instrument chirurgical selon la revendication 9, comprenant en outre au moins une entretoise (700) positionnée entre l'arbre et le tube externe.

11. Instrument chirurgical selon la revendication 1, comprenant en outre un raccord souple positionné au moins en partie entre le corps et le tube externe.

12. Instrument selon la revendication 1, dans lequel le tube externe est plus souple que l'arbre par rapport au corps.

13. Instrument chirurgical selon la revendication 1, dans lequel la résistance à la déformation du tube externe par une force appliquée latéralement au niveau d'un point longitudinal donné est inférieure d'au moins 10 % à la résistance à la déformation de l'arbre par la même force au niveau du même point longitudinal.

14. Instrument chirurgical selon la revendication 13, dans lequel la résistance à la déformation du tube externe par une force appliquée latéralement au niveau d'un point longitudinal donné est inférieure d'au moins 50 % à la résistance à la déformation de l'arbre par la même force au niveau du même point longitudinal.

15. Instrument chirurgical selon la revendication 14, dans lequel la résistance à la déformation du tube externe par une force appliquée latéralement au niveau d'un point longitudinalement donné est inférieure d'au moins 90 % à la résistance à la déformation de l'arbre par la même force au niveau du même point longitudinal.

16. Instrument chirurgical selon l'une quelconque des revendications précédentes, dans lequel la souplesse du tube externe au niveau d'un premier point longitudinal est différente de la souplesse du tube externe au niveau d'un second point longitudinal.

17. Instrument chirurgical selon l'une quelconque des revendications précédentes, dans lequel un diamètre radial maximal du composant venant en contact avec du tissu est plus petit qu'un diamètre radial minimal de l'arbre.

18. Instrument chirurgical selon l'une quelconque des revendications 1 à 17, dans lequel un diamètre radial maximal du composant venant en contact avec du tissu est approximativement identique à un diamètre radial maximal de l'arbre.

19. Instrument chirurgical selon l'une quelconque dés revendications précédentes, dans lequel un diamètre intérieur du tube externe (100) dans une région adjacente à l'arbre est plus petit qu'un diamètre intérieur du tube externe dans une région adjacente au composant venant en contact avec du tissu.

20. Instrument chirurgical selon la revendication 11, dans lequel le raccord souple est une gaine élastique.

21. Instrument chirurgical selon l'une quelconque des revendications précédentes, dans lequel le tube externe entoure au moins une partie du composant venant en contact avec du tissu.
